(19) Europäisches Patentamt
European Patent Office
Office européen des brevets

(11) Numéro de publication: **0 044 768**
**B1**

(12) # FASCICULE DE BREVET EUROPEEN

(45) Date de publication du fascicule du brevet: **04.05.83**

(51) Int. Cl.³: **C 07 D 231/22**

(21) Numero de dépôt: **81401085.6**

(22) Date de dépôt: **07.07.81**

(54) **Procédé de préparation de phényl-1 carbamoyl-3 pyrazolones-5.**

(30) Priorité: **18.07.80 FR 8015878**

(43) Date de publication de la demande:
**27.01.82 Bulletin 82/4**

(45) Mention de la délivrance du brevet:
**04.05.83 Bulletin 83/18**

(84) Etats contractants désignés:
**BE CH DE FR GB IT LI NL**

(56) Documents cités:
**FR-A-944 617**
**US-A-2 153 615**
**BEILSTEINS HANDBUCH DER ORGANISCHEN CHEMIE, IV, vol. 25, 1936, Berlin, DE »Heterocyclische Reihe«, »F. Oxocarbonsäuren«, pages 204—218**
**JOURNAL OF THE AMERICAN CHEMICAL SOCIETY, vol. 64, septembre 1942 US WEISSBERGER et al.: »Investig ation of pyrazole compounds. I. The reaction product of phenylhydrazine and ethyl cyanoacetate«, pages 2133—2136**

**Le dossier contient des informations techniques présentées postérieurement au dépôt de la demande et ne figurant pas dans le présent fascicule.**

(73) Titulaire: **P C U K PRODUITS CHIMIQUES UGINE KUHLMANN, Service Propriété Industrielle Tour Manhattan, F-92087 Paris La Défense 2 Cédex 21 (FR)**

(72) Inventeur: **Breda, Antoine Georges Léon Jacques, 15 Parc des Hauts Prés Saint Genis Les Ollieres, F-69290 Craponne (FR)**
Inventeur: **Roussel, Jacques, Lotissement Chevalier, F-27670 Bosc Roger en Roumois (FR)**

(74) Mandataire: **Leboulenger, Jean et al, P C U K PRODUITS CHIMIQUES UGINE KUHLMANN Service Propriéte Industrielle Tour Manhattan, F-92087 Paris La Défense 2 Cedex 21 (FR)**

**EP 0 044 768 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

BUNDESDRUCKEREI BERLIN

## Procédé de préparation de phényl-1 carbamoyl-3 pyrazolones-5

La présente invention concerne la préparation des phényl-1 carbamoyl-3 pyrazolones-5 de formule générale I

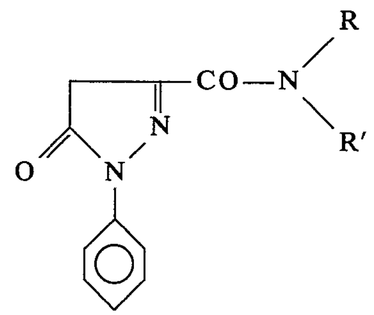

(I)

dans laquelle les deux symboles R et R', identiques ou différents, représentent chacun un atome d'hydrogène ou un radical alkyle contenant 1 à 4 atomes de carbone et éventuellement substitué, par exemple par un groupe hydroxy.

Ces composés connus, utiles comme copulants pour la préparation de colorants monoazoïques de dispersion pour fibres hydrophobes ou celle de colorants complexes chromifères pour fibres de polyamides naturels ou synthétiques, sont habituellement préparés par condensation de la phénylhydrazine avec l'oxalacétate de diéthyle, cyclisation de l'hydrazone formée en phényl-1 carbéthoxy-3 pyrazolone-5 et amidification, selon le schéma réactionnel suivant:

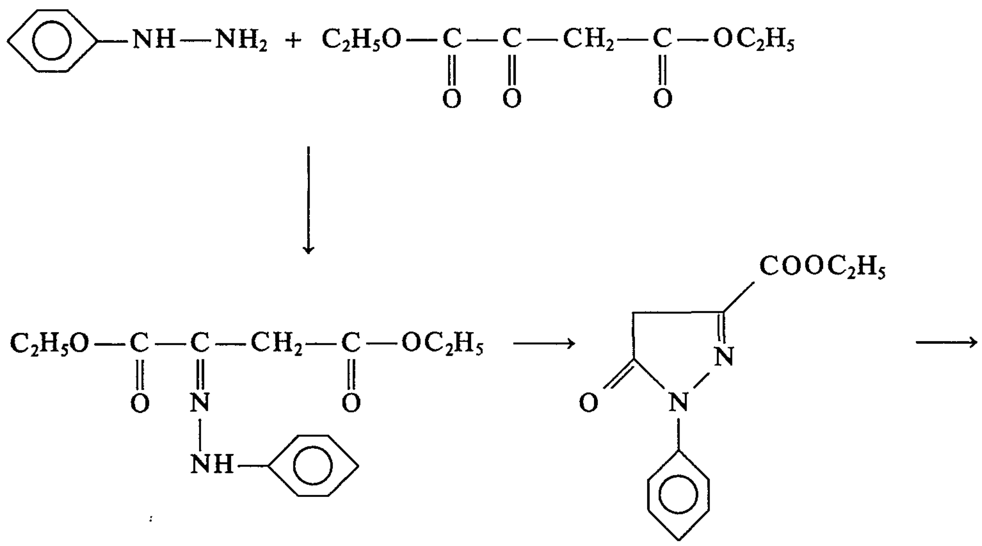

(I)

(II) (III)

Ce mode de préparation présente cependant plusieurs inconvénients en pratique industrielle. En effet, à partir des pâtes aqueuses de phényl-1 carbéthoxy-3 pyrazolone-5 III, on ne peut pas éviter lors de l'amidification une hydrolyse partielle de groupe carbéthoxy en groupe carboxy, ce qui est préjudiciable au rendement et à la qualité du produit final. D'autre part, les réactions s'effectuent en plusieurs étapes avec des isolements intermédiaires préjudiciables au rendement et à la productivité.

Il a maintenant été trouvé qu'on peut minimiser l'hydrolyse partielle du groupe carbéthoxy, donc la formation de phényl-1 carboxy-3 pyrazolone-5, et obtenir dans un seul appareil une phényl-1 carbamoyl-3 pyrazolone-5 de formule I pratiquement pure si l'on chauffe une pâte aqueuse de l'hydrazone de formule II sans dépasser 85°C, de préférence 80°C, jusqu'à la fusion totale de l'hydrazone, puis élimine la phase aqueuse et traite l'hydrazone anhydre restante avec 2 à 12 équivalents-molaires d'un composé de formule IV

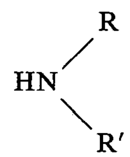

(IV)

où R et R′ ont les mêmes significations que ci-dessus, à une température comprise entre 20 et 100° C, de préférence entre 80 et 90° C.

Le chauffage de la pâte aqueuse d'hydrazone de formule II, éventuellement diluée à l'eau, provoque une séparation en deux phases du mélange réactionnel, l'hydrazone fondue constituant la couche inférieure. Si, pour des raisons de commodité, on désire éliminer la phase aqueuse par le bas du réacteur, il suffit d'ajouter au mélange réactionnel une quantité de sel suffisante pour provoquer une inversion de phases.

La cyclisation de l'hydrazone de formule II en phényl-1 carbéthoxy-3 pyrazolone-5 de formule III et l'amidification de cette dernière en phényl-1 carbamoyl-3 pyrazolone-5 de formule I sont réalisées pratiquement en un seul stade lors du traitement par le composé de formule IV. Pour éviter une prise en masse lors de ce traitement, on peut diluer le mélange réactionnel avec de l'alcool avant ou pendant le traitement. La réaction terminée, on ajoute de l'eau ce qui permet, après élimination de l'alcool, de filtrer éventuellement la pyrazolone de formule I qui a précipité; on peut aussi avantageusement ajouter un alcali à la suspension aqueuse de façon à solubiliser la pyrazolone de formule I, la solution obtenue étant directement utilisabel pour la fabrication ultérieure de colorants azoïques.

Avec certaines amines liquides de formule IV, notamment la monoéthanolamine, il n'est pas nécessaire de diluer avec de l'alcool; il convient cependant dans ce cas d'utiliser au moins 3 équivalents-molaires d'amine.

Les exemples suivants illustrent l'invention sans la limiter. Les parties et pourcentages indiqués s'entendent en poids, sauf mention contraire.

Exemple 1

Dans un réacteur en acier vitrifié d'une contenance de 7 000 parties en volume, on charge 1 000 parties d'eau, puis 4 000 parties d'une pâte aqueuse de phénylhydrazone d'oxalacétate de diéthyle contenant environ 44% de matières sèches et 1 347 parties d'hydrazone pure. On ajoute ensuite 750 parties de chlorure de sodium pour régler la densité de la phase aqueuse à environ 1,18, chauffe le mélange à 80° C jusqu'à fusion totale de l'hydrazone surnageante, puis élimine la phase aqueuse salée.

Dans l'hydrazone anhydre restante, maintenue entre 70 et 80° C, on coule 1 050 parties de monoéthanolamine; la réaction étant exothermique, l'introduction de monoéthanolamine est réglée de telle sorte que la température reste inférieure à 85° C. Lorsque l'introduction de monoéthanolamine est terminée, on chauffe progressivement, tout en distillant l'alcool éthylique formé; la température de la masse réactionnelle en fin de distillation ne doit pas dépasser 110° C.

On obtient ainsi 2 280 parties d'un liquide visqueux contenant 1 185 parties de phényl-1 N-($\beta$-hydroxyéthyl) carbamoyl-3 pyrazolone-5. Ce liquide visqueux peut être éventuellement dilué à l'eau et est directement utilisable pour la fabrication de colorants azoïques.

Exemple 2

On opère comme à l'exemple 1, sauf qu'on remplace la monoéthanolamine par 1 696 parties de diéthanolamine.

On obtient un liquide visqueux contenant 1 367 parties de phényl-1 N,N-bis($\beta$-hydroxyéthyl)-carbamoyl-3 pyrazolone-5.

**Revendications**

1. Procédé de préparation d'une phényl-1 carbamoyl-3 pyrazolone-5 de formule I

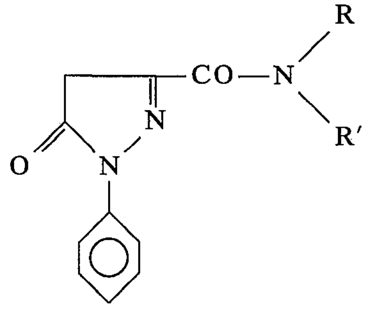

(I)

dans laquelle les deux symboles R et R', identiques ou différents, représentent chacun un atome d'hydrogène ou un radical alkyle contenant 1 à 4 atomes de carbone et éventuellement substitué, caractérisé en ce que l'on chauffe sans dépasser 85°C une pâte aqueuse de phénylhydrazone d'oxalacétate de diéthyle jusqu'à la fusion totale de cette hydrazone, puis on élimine la phase aqueuse et traite l'hydrazone restante avec 2 à 12 équivalents-molaires d'un composé de formule IV

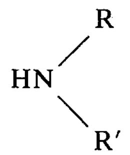

(IV)

où R et R' ont les mêmes significations que ci-dessus, à une température comprise entre 20 et 100°C.

2. Procédé selon la revendication 1 dans lequel le traitement final est effectué à une température comprise entre 80 et 90°C.

3. Procédé selon la revendication 1 ou 2 dans lequel le traitement final est effectué avec addition d'alcool au mélange réactionnel.

4. Procédé selon la revendication 1 ou 2 pour la préparation de la phényl-1 N-($\beta$-hydroxyéthyl) carbamoyl-3 pyrazolone-5, dans lequel le composé de formule IV est la monoéthanolamine employée à raison d'au moins 3 équivalents-molaires.

## Patentansprüche

1. Verfahren zur Herstellung eines 1-Phenyl-3-carbamoyl-5-pyrazolons der Formel I

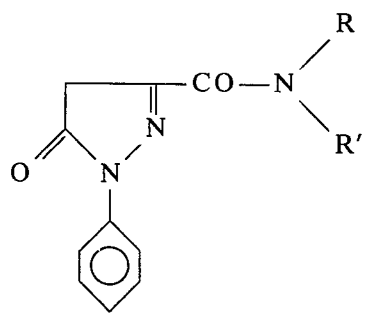

(I)

in der die beiden Symbole R und R', die gleichartig oder verschieden sind, jeweils ein Wasserstoffatom oder eine gegebenenfalls substituierte Alkylgruppe mit 1 bis 4 Kohlenstoffatomen bedeuten, dadurch gekennzeichnet, daß man einen wäßrigen Brei des Phenylhydrazons von Oxalessigsäurediäthylester ohne 85°C zu übersteigen bis zum vollständigen Schmelzen dieses Hydrazons erhitzt, dann die wäßrige Phase entfernt und das verbleibende Hydrazon mit 2 bis 12 Moläquivalenten einer Verbindung der Formel IV

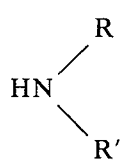

(IV)

in der R und R' die oben angegebenen Bedeutungen besitzen, bei einer Temperatur zwischen 20 und 100°C behandelt.

2. Verfahren nach Anspruch 1, dadurch gekennzeichnet, daß die Endbehandlung bei einer Temperatur zwischen 80 und 90°C durchgeführt wird.

3. Verfahren nach Anspruch 1 oder 2, dadurch gekennzeichnet, daß die Endbehandlung unter Zugabe von Alkohol zu der Reaktionsmischung durchgeführt wird.

4. Verfahren nach Anspruch 1 oder 2 zur Herstellung von 1-Phenyl-N-($\beta$-hydroxyäthyl)-3-carbamoyl-5-pyrazolon, dadurch gekennzeichnet, daß man als Verbindung der Formel IV das Monoäthanolamin in einer Menge von mindestens 3 Moläquivalenten einsetzt.

## Claims

1. Process for the preparation of a 1-phenyl-3-carbamoyl-pyrazol-5-one of the formula I

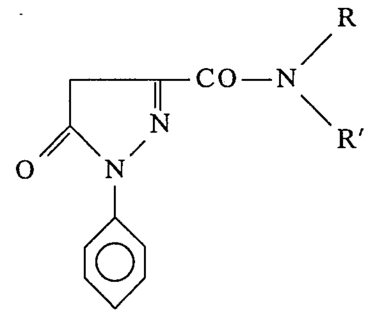

(I)

in which both symbols R and R′ are the same or different and each represents a hydrogen atom or an alkyl radical containing 1 to 4 carbon atoms and possibly substituted, characterized in that an aqueous paste of diethyl oxalacetate phenylhydroazone is heated without exceeding 85° C until said hydrazone is completely melted, then the aqueous phase is removed and the remaining hydrazone is treated with 2 to 12 molar equivalents of a compound of the formula IV

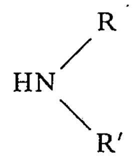

(IV)

wherein R and R′ have the same significances as above, at a temperature between 20 and 100° C.

2. Process according to claim 1 in which the final treatment is effected at a temperature between 80 and 90° C.

3. Process according to claim 1 or 2 in which the final treatment is effected with addition of alcohol to the reaction mixture.

4. Process according to claim 1 or 2 for the preparation of 1-phenyl-N-(β-hydroxyethyl)-3-carbamoyl-pyrazol-5-one, in which the compound of formula IV is monoethanolamine used at the rate of at least 3 molar equivalents.